# EUROPEAN PATENT APPLICATION

(11) **EP 4 137 133 A1**
(43) Date of publication of application: **22.02.2023**
(21) Application number: 21192130.9
(22) Date of filing: 19.08.2021
(51) Int. Cl.: A61K 31/44, C07D 211/60, A61P 9/10, A61P 13/12, A61P 29/00

(54) **CRYSTALLINE FORM OF AVACOPAN**

(71) Applicant: Sandoz AG, 4056 Basel (CH)
(72) Inventor: The designation of the inventor has not yet been filed
(74) Representative: Kluschanzoff, Harald

(57) **Abstract**

The present invention relates to a crystalline form of avacopan, to a process for its preparation and its use for pharmaceutical purposes.

## Description

### FIELD OF THE INVENTION

The present invention relates to a crystalline form of avacopan, a process for its preparation and its use for pharmaceutical purposes.

### BACKGROUND OF THE INVENTION

Avacopan, an orally administered antagonist of the Complement 5a receptor (C5aR), is being studied for the treatment of anti-neutrophil cytoplasmic antibody (ANCA)-associated vasculitis (AAV). The chemical name of avacopan is (2*R*,3*S*)-2-(4-(cyclopentylamino)phenyl)-1-(2-fluoro-6-methylbenzoyl)-*N*-(4-methyl-3-(trifluoromethyl)phenyl)piperidine-3-carboxamide and it can be represented by the following chemical structure according to Formula (A)

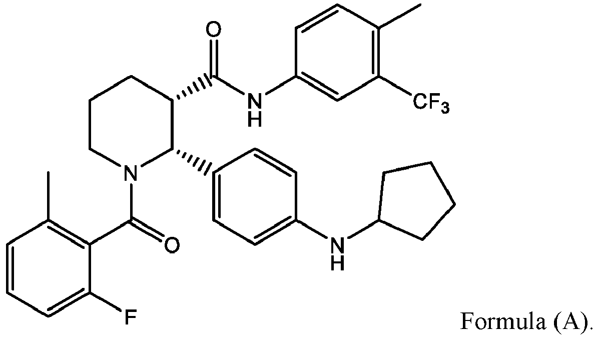

The preparation of avacopan is described in WO 2016/053890 A1. Avacopan is obtained as off-white crystals in example 1 and as colorless crystals in example 2, respectively.

WO 2021/092292 A1 discloses a crystalline form of avacopan characterized by powder X-ray diffraction and differential scanning calorimetry. The same crystalline form is reported in WO 2021/092286 A1.

Different solid-state forms of an active pharmaceutical ingredient often possess different properties. Differences in physicochemical properties of solid-state forms can play a crucial role for the improvement of pharmaceutical compositions, for example, pharmaceutical formulations with improved dissolution profile and bioavailability or with improved stability or shelf-life can become accessible due to an improved solid-state form of an active pharmaceutical ingredient. Also processing or handling of the active pharmaceutical ingredient during the formulation process may be improved. New solid-state forms of an active pharmaceutical ingredient can thus have desirable processing properties. They can be easier to handle, better suited for storage, and/or allow for better purification, compared to previously known solid forms.

The thermodynamically most stable polymorph of an active pharmaceutical ingredient is often used for the preparation of a drug product, since phase transitions during pharmaceutical standard processes like compaction, milling and wet granulation can be reduced to the extent possible. However, a huge drawback connected with the thermodynamically most stable form of a drug substance is the fact, that it is the least soluble form, which translates into decreased bioavailability. This is especially critical for low soluble compounds like avacopan. Therefore, using a metastable form of a drug substance is sometimes desirable on account of its special properties, in particular higher solubility and hence bioavailability. However, it is important to avoid any possible solid form transitions, which may occour for a metastable modification during pharmaceutical processing or storage, since this can have a huge impact on the safety and efficacy of a drug product. Therefore, not every metastable form of an active pharmaceutical ingredient can be used for the production of a pharmaceutical composition.

There is thus a need for the provision of solid-state forms of avacopan having improved physicochemical properties.

In particular, there is a need to provide an improved crystalline form of avacopan, which is kinetically stable *e.g.* does not undergo phase transformations during standard pharmaceutical processing and storage and at the same time shows high solubility and dissolution rates.

### SUMMARY OF THE INVENTION

The present invention provides a crystalline form of avacopan, which is hereinafter also designated as "Form HyA".

It is noteworthy, that in the course of a comprehensive polymorph screening program, Form HyA of the present invention was only obtained from a single experiment under very specific crystallization conditions (see Example 1 hereinafter).

Form HyA of the present invention possesses one or more unexpected improved physicochemical properties selected from the group consisting of dissolution rate, solubility, chemical stability, physical stability, chemical purity, residual solvent content, hygroscopicity, melting point, morphology, flowability, wettability, bulk density and compressibility.

For example, Form HyA of the present invention is physically stable against temperature and humidity stress which is relevant for the manufacture and storage of a pharmaceutical composition comprising avacopan Form HyA (see Examples 6 to 8 hereinafter).

In addition, Form HyA of the present invention is more soluble in physiologically relevant media such as 0.1N HCl solution compared to the known crystalline form of avacopan disclosed in WO 2021/092292 A1 (see Comparative Example 1 hereinafter) which is a crucial advantage for a low soluble drug substance like avacopan in order to reach the desired *in vivo* blood levels. Form HyA of the present invention is therefore the preferred solid form of avacopan for the preparation of a pharmaceutical composition.

**Abbreviations**

| | |
|---|---|
| PXRD | powder X-ray diffractogram |
| DSC | differential scanning calorimetry |
| TGA | thermogravimetric analysis |
| GMS | gravimetric moisture sorption |
| RH | relative humidity |
| RT | room temperature |
| w-% | weight percent |

### Definitions

In the context of the present invention the following definitions have the indicated meaning, unless explicitly stated otherwise:
As used herein, the term "measured at a temperature in the range of from 20 to 30°C" refers to a measurement under standard conditions. Typically, standard conditions mean a temperature in the range of from 20 to 30°C, *i.e.* at room temperature. Standard conditions can mean a temperature of about 22°C. Typically, standard conditions can additionally mean a measurement under 20-60% RH, preferably 30-50% RH, more preferably 40% RH

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order *e.g*. extends over approximately 100 to 1000 atoms, whereas short-range order is over a few atoms only (see *"*Fundamentals of Powder Diffraction and Structural Characterization of Materials " by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 3.7° 2-Theta for example can appear between 3.5° and 3.9° 2-Theta, preferably between 3.6° and 3.8° 2-Theta on most X-ray diffractometers under standard conditions. Furthermore, one skilled in the art will appreciate that relative reflection intensities will show inter-apparatus variability as well as variability due to degree of crystallinity, preferred orientation, particle size, sample preparation and other factors known to those skilled in the art and should be taken as qualitative measure only.

Crystalline Form HyA of avacopan of the present invention may be referred to herein as being characterized by graphical data "as shown in" a figure. Such data include, for example, powder X-ray diffraction. The person skilled in the art understands that factors such as variations in instrument type, response and variations in sample directionality, sample concentration and sample purity may lead to small variations for such data when presented in graphical form, for example variations relating to the exact reflection positions and intensities. However, a comparison of the graphical data in the figures herein with the graphical data generated for another or an unknown solid form and the confirmation that two sets of graphical data relate to the same crystal form is well within the knowledge of a person skilled in the art.

The term"solid-state form" as used herein refers to any crystalline and/or amorphous phase of a compound.

As used herein, the term "amorphous" refers to a solid-state form of a compound that is not crystalline. An amorphous compound possesses no long-range order and does not display a definitive X-ray diffraction pattern with reflections.

### BRIEF DESCRIPTION OF THE FIGURES

**Figure 1****:** illustrates a representative PXRD of Form HyA of avacopan of the present invention. The x-axis shows the scattering angle in °2-Theta, the y-axis shows the intensity of the scattered X-ray beam in counts of detected photons.
**Figure 2****:** illustrates a representative DSC curve of Form HyA of avacopan according to the present invention. The x-axis shows the temperature in degree Celsius (°C), the y-axis shows the heat flow rate in Watt per gram (W/g) with endothermic peaks going up.
**Figure 3****:** illustrates a representative TGA curve of Form HyA of avacopan according to the present invention. The x-axis shows the temperature in degree Celsius (°C), the y-axis shows the mass (loss) of the sample in weight percent (w-%).
**Figure 4****:** illustrates representative GMS isotherms of Form HyA of avacopan according to the present invention in the range of from 0 to 90% relative humidity. The x-axis displays the relative humidity in percent (%) measured at a temperature of (25.0 ± 0.1) °C, the y-axis displays the mass changes in weight percent (w-%). The sample weight at 0% relative humidity is used as reference weight. The sorption curve points are displayed in triangles, desorption curve points as squares.
**Figure 5****:** displays the dissolution curves of the crystalline form of avacopan disclosed in WO 2021/092292 A1 (black squares) and of the crystalline Form HyA of avacopan of the present invention (black triangles) in 0.1N HCl measured at 37°C. The x-axis shows the time in hours, the y-axis the avacopan concentration of the solution in microgram per millilitre.

### DETAILED DESCRIPTION OF THE INVENTION

The present invention provides a crystalline form of avacopan, herein also designated as "Form HyA".

Crystalline Form HyA of avacopan of the present invention may be characterized by analytical methods well known in the field of the pharmaceutical industry for characterizing solids. Such methods comprise but are not limited to powder X-ray diffraction, DSC; TGA and GMS. Avacopan Form HyA of the present invention may be characterized by one of the aforementioned analytical methods or by combining two or more of them. In particular, Form HyA of avacopan of the present invention may be characterized by any one of the following embodiments or by combining two or more of the following embodiments.

In one embodiment the invention relates to a crystalline form (Form HyA) of avacopan characterized by having a PXRD comprising reflections at 2-Theta angles of:
(3.7 ± 0.2)°, (6.1 ± 0.2)° and (17.4 ± 0.2)°; or
(3.7 ± 0.2)°, (6.1 ± 0.2)°, (8.9 ± 0.2)° and (17.4 ± 0.2)°; or
(3.7 ± 0.2)°, (6.1 ± 0.2)°, (8.9 ± 0.2)°, (11.1 ± 0.2)° and (17.4 ± 0.2)°; or (3.7 ± 0.2)°, (6.1 ± 0.2)°, (8.9 ± 0.2)°, (11.1 ± 0.2)°, (12.1 ± 0.2)° and (17.4 ± 0.2)°; or (3.7 ± 0.2)°, (6.1 ± 0.2)°, (8.9 ± 0.2)°, (11.1 ± 0.2)°, (12.1 ± 0.2)°, (14.8 ± 0.2)° and (17.4 ± 0.2)°; or
(3.7 ± 0.2)°, (6.1 ± 0.2)°, (8.9 ± 0.2)°, (11.1 ± 0.2)°, (12.1 ± 0.2)°, (14.8 ± 0.2)°, (15.9 ± 0.2)° and (17.4 ± 0.2)°; or
(3.7 ± 0.2)°, (6.1 ± 0.2)°, (8.9 ± 0.2)°, (11.1 ± 0.2)°, (12.1 ± 0.2)°, (14.8 ± 0.2)°, (15.6 ± 0.2)°, (15.9 ± 0.2)° and (17.4 ± 0.2)°; or
(3.7 ± 0.2)°, (6.1 ± 0.2)°, (8.9 ± 0.2)°, (9.8 ± 0.2)°, (11.1 ± 0.2)°, (12.1 ± 0.2)°, (14.8 ± 0.2)°, (15.6 ± 0.2)°, (15.9 ± 0.2)° and (17.4 ± 0.2)°;
when measured at a temperature in the range of from 20 to 30°C with Cu-Kalpha_{1,2} radiation having a wavelength of 0.15419 nm.

In another embodiment, the invention relates to a crystalline form (Form HyA) of avacopan characterized by having a PXRD comprising reflections at 2-Theta angles of (8.9 ± 0.2)°, (9.8 ± 0.2)°, (12.1 ± 0.2)°, (14.8 ± 0.2)°, (15.6 ± 0.2)°, (15.9 ± 0.2)°, (17.4 ± 0.2)°, (19.4 ± 0.2)°, (19.5 ± 0.2)° and (22.4 ± 0.2)°, when measured at a temperature in the range of from 20 to 30°C with Cu-Kalpha_{1,2} radiation having a wavelength of 0.15419 nm.

In a further embodiment, the present invention relates to a crystalline form (Form HyA) of avacopan characterized by having a PXRD comprising reflections at 2-Theta angles of:
(3.7 ± 0.1)°, (6.1 ± 0.1)° and (17.4 ± 0.1)°; or
(3.7 ± 0.1)°, (6.1 ± 0.1)°, (8.9 ± 0.1)° and (17.4 ± 0.1)°; or (3.7 ± 0.1)°, (6.1 ± 0.1)°, (8.9 ± 0.1)°, (11.1 ± 0.1)° and (17.4 ± 0.1)°; or (3.7 ± 0.1)°, (6.1 ± 0.1)°, (8.9 ± 0.1)°, (11.1 ± 0.1)°, (12.1 ± 0.1)° and (17.4 ± 0.1)°; or (3.7 ± 0.1)°, (6.1 ± 0.1)°, (8.9 ± 0.1)°, (11.1 ± 0.1)°, (12.1 ± 0.1)°, (14.8 ± 0.1)° and (17.4 ± 0.1)°; or
(3.7 ± 0.1)°, (6.1 ± 0.1)°, (8.9 ± 0.1)°, (11.1 ± 0.1)°, (12.1 ± 0.1)°, (14.8 ± 0.1)°, (15.9 ± 0.1)° and (17.4 ± 0.1)°; or
(3.7 ± 0.1)°, (6.1 ± 0.1)°, (8.9 ± 0.1)°, (11.1 ± 0.1)°, (12.1 ± 0.1)°, (14.8 ± 0.1)°, (15.6 ± 0.1)°, (15.9 ± 0.1)° and (17.4 ± 0.1)°; or
(3.7 ± 0.1)°, (6.1 ± 0.1)°, (8.9 ± 0.1)°, (9.8 ± 0.1)°, (11.1 ± 0.1)°, (12.1 ± 0.1)°, (14.8 ± 0.1)°, (15.6 ± 0.1)°, (15.9 ± 0.1)° and (17.4 ± 0.1)°;
when measured at a temperature in the range of from 20 to 30°C with Cu-Kalpha_{1,2} radiation having a wavelength of 0.15419 nm.

In yet another embodiment, the invention relates to a crystalline form (Form HyA) of avacopan characterized by having a PXRD comprising reflections at 2-Theta angles of (8.9 ± 0.1)°, (9.8 ± 0.1)°, (12.1 ± 0.1)°, (14.8 ± 0.1)°, (15.6 ± 0.1)°, (15.9 ± 0.1)°, (17.4 ± 0.1)°, (19.4 ± 0.1)°, (19.5 ± 0.1)° and (22.4 ± 0.1)°, when measured at a temperature in the range of from 20 to 30°C with Cu-Kalpha_{1,2} radiation having a wavelength of 0.15419 nm.

The PXRD of avacopan Form HyA of the present invention can be readily distinguished from the PXRD of the crystalline avacopan form disclosed in WO 2021/092292 A1. For example, the PXRD of Form HyA of the present invention shows reflections at 2-Theta angles of (3.7 ± 0.2)°, (6.1 ± 0.2)° and (7.4 ± 0.2)°, whereas the crystalline form disclosed in WO 2021/092292 A1 shows no reflections at these positions. On the other hand, the crystalline form disclosed in WO 2021/092292 A1 shows characteristic reflections at 2-Theta angles of (8.1 ± 0.2)° and (8.4 ± 0.2)°, *e.g.* at positions where Form HyA of the present invention possesses no reflections.

Hence, in one embodiment the present invention relates to a crystalline form (Form HyA) of avacopan characterized by having a PXRD comprising reflection at 2-Theta angles of (3.7 ± 0.2)°, (6.1 ± 0.2)° and/or (7.4 ± 0.2)°, when measured at a temperature in the range of from 20 to 30°C with Cu-Kalpha_{1,2} radiation having a wavelength of 0.15419 nm.

In another embodiment the present invention relates to a crystalline form (Form HyA) of avacopan characterized by having a PXRD comprising reflection at 2-Theta angles of (3.7 ± 0.1)°, (6.1 ± 0.1)° and/or (7.4 ± 0.1)°, when measured at a temperature in the range of from 20 to 30°C with Cu-Kalpha_{1,2} radiation having a wavelength of 0.15419 nm.

In still another embodiment, the present invention relates to a crystalline form (Form HyA) of avacopan as defined in any one of the above described embodiments characterized by having a PXRD comprising no reflection at 2-Theta angles of (8.1 ± 0.2)° and (8.4 ± 0.2)°, when measured at a temperature in the range of from 20 to 30°C with Cu-Kalpha_{1,2} radiation having a wavelength of 0.15419 nm.

In a particular preferred embodiment, the present invention relates to a crystalline form (Form HyA) of avacopan characterized by having a powder X-ray diffractogram comprising reflections at 2-Theta angles of (3.7 ± 0.2)°, (6.1 ± 0.2)° and/or (7.4 ± 0.2)°, but comprising no reflection at 2-Theta angles of (8.1 ± 0.2)° and (8.4 ± 0.2)°, when measured at a temperature in the range of from 20 to 30°C with Cu-Kalpha_{1,2} radiation having a wavelength of 0.15419 nm.

In another preferred embodiment, the present invention relates to a crystalline form (Form HyA) of avacopan characterized by having a powder X-ray diffractogram comprising reflections at 2-Theta angles of (3.7 ± 0.1)°, (6.1 ± 0.1)° and/or (7.4 ± 0.1)°, but comprising no reflection at 2-Theta angles of (8.1 ± 0.2)° and (8.4 ± 0.2)°, when measured at a temperature in the range of from 20 to 30°C with Cu-Kalpha_{1,2} radiation having a wavelength of 0.15419 nm.

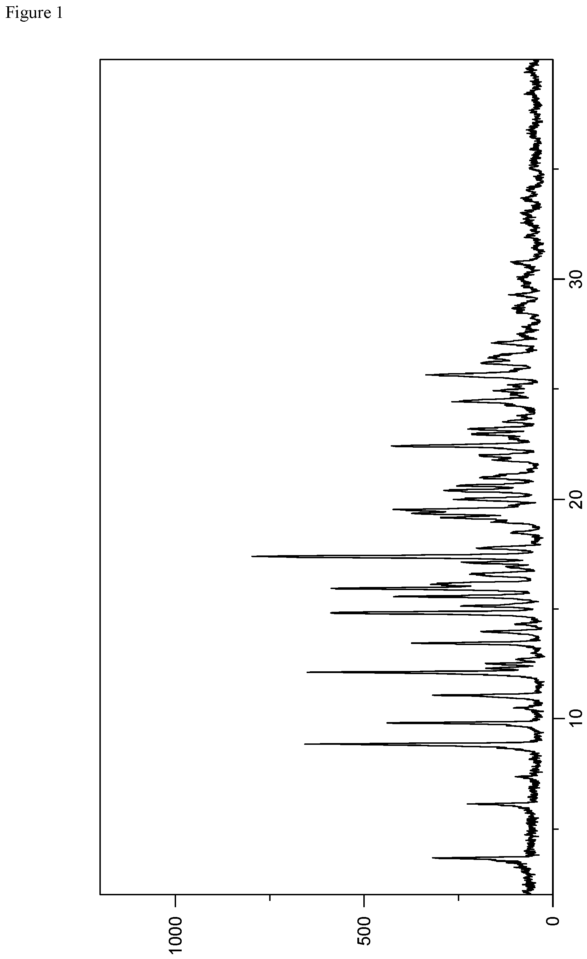

In yet another embodiment, the present invention relates to a crystalline form (Form HyA) of avacopan characterized by having a PXRD essentially the same as shown in Figure 1 of the present invention, when measured at a temperature in the range of from 20 to 30°C with Cu-Kalpha_{1,2} radiation having a wavelength of 0.15419 nm.

In a further embodiment, the present invention relates to a crystalline form (Form HyA) of avacopan characterized by having a DSC curve comprising an endothermic peak, preferably a first endothermic peak, having an onset at a temperature of (120 ± 5)°C, preferably of (120 ± 3)°C, more preferably of (120 ± 2)°C and most preferably of (120 ± 1)°C, when measured at a heating rate of 10 K/min.

In another embodiment, the present invention relates to a crystalline form (Form HyA) of avacopan characterized by having a DSC curve comprising an endothermic peak, preferably a first endothermic peak, having a maximum at a temperature of (122 ± 5)°C, preferably of (122 ± 3)°C, more preferably of (122 ± 2)°C and most preferably of (122 ± 1)°C, when measured at a heating rate of 10 K/min.

In one embodiment, the present invention relates to a crystalline form (Form HyA) of avacopan characterized by having a TGA curve showing a mass loss of not more than about 3.3 w-%, preferably of about 3.3 w-%, when heated from 25 to 130°C at a rate of 10 K/min.

In an additional embodiment, the present invention relates to a crystalline form (Form HyA) of avacopan characterized by showing a mass change of not more than about 1.0 w-%, preferably of about 1.0 w-% based on the weight of the crystalline form, when measured with GMS at a relative humidity in the range of from 15 to 90% and a temperature of (25.0 ± 0.1)°C.

In another embodiment, the present invention relates to a crystalline form (Form HyA) of avacopan characterized by showing a mass change of not more than about 3.2 w-%, preferably of about 3.2 w-% based on the weight of the crystalline form, when measured with GMS at a relative humidity in the range of from 0 to 90% and a temperature of (25.0 ± 0.1)°C.

In a further aspect, the present invention relates to a process for the preparation of the crystalline form (Form HyA) of avacopan as defined in any one of the above described aspects and their corresponding embodiments comprising:
(a) dissolving avacopan in methanol;
(b) optionally filtering the solution;
(c) optionally adding avacopan Form HyA seed crystals to the solution obtained in (a) or (b);
(d) crystallizing avacopan from the solution obtained in (a) or (b) or from the mixture obtained in (c);
(e) separating at least a part of the crystals obtained in (d) from the mother liquor;
(f) optionally washing the isolated crystals obtained in (e); and
(g) drying the crystals obtained in any one of steps (d) to (f).

In another aspect, the present invention relates to the use of the crystalline form (Form HyA) of avacopan as defined in any one of the above described aspects and their corresponding embodiments for the preparation of a pharmaceutical composition comprising avacopan. The pharmaceutical composition is preferably an oral dosage form *e.g.* selected from the group consisting of a tablet, a capsule or a suspension, most preferably the pharmaceutical composition is a hard gelatin capsule.

In a further aspect, the present invention relates to a pharmaceutical composition comprising the crystalline form (Form HyA) of avacopan as defined in any one of the above described aspects and their corresponding embodiments, preferably in a predetermined and/or effective amount, and at least one pharmaceutically acceptable excipient.

Preferably, the predetermined and/or effective amount of the crystalline form (Form HyA) of avacopan as defined in any one of the above described aspects and their corresponding embodiments is in the range of from 10 to 60 mg calculated as anhydrous avacopan. For example, the predetermined and/or effective amount is selected from the group consisting of 10 mg, 20 mg, 30 mg, 40 mg, 50 mg and 60 mg calculated as anhydous avacopan.

In one embodiment, the pharmaceutical composition comprising the crystalline form (Form HyA) of avacopan as defined above is an oral dosage form, preferably a capsule, a tablet or a suspension, most preferably a hard gelatin capsule.

In a further aspect the invention relates to the crystalline form (Form HyA) of avacopan or the pharmaceutical composition comprising the crystalline form (Form HyA) of avacopan as defined in any one of the above described aspects and their corresponding embodiments for use as a medicament.

In yet another aspect the invention relates to the crystalline form (Form HyA) of avacopan or the pharmaceutical composition comprising the crystalline form (Form HyA) of avacopan as defined in any one of the above described aspects and their corresponding embodiments for use in the treatment of anti-neutrophil cytoplasmic antibody (ANCA)-associated vasculitis (AAV).

In an alternative embodiment the invention concerns a method of treating anti-neutrophil cytoplasmic antibody (ANCA)-associated vasculitis (AAV) said method comprising administering an effective amount of the crystalline form (Form HyA) of avacopan or the pharmaceutical composition comprising the crystalline form (Form HyA) of avacopan as defined in any one of the above described aspects and their corresponding embodiments to a patient in need of such a treatment.

### EXAMPLES

The following non-limiting examples are illustrative for the disclosure and are not to be construed as to be in any way limiting for the scope of the invention.

### Example 1: Solid form screening

A comprehensive solid form screening program was conducted using a variety of different solvents and solvent/water mixtures, respectively. Thereby, amorphous avacopan was applied as starting material. The amorphous material was prepared by freeze drying a solution of avacopan (e.g. be prepared according to example 1 or 2 of WO 2016/053890 A1) in a mixture of acetonitrile and H₂O (3/1, v/v).

To the thus obtained amorphous avacopan (48-50 mg) 1 mL of solvent or solvent mixture according to table 1 was added at RT. Obtained solutions were left at RT in closed vials without stirring. If no solution was obtained heat was applied and the thus obtained solutions were again allowed to stand at RT without stirring. If still no clear solution was obtained, the suspensions were stirred at RT in closed vials. In case no crystals deposited upon standing at RT, the solutions were store in a fridge at 4-8°C. If still no crystals were observed upon storage in the fridge, the solvents were allowed to evaporate in open vials or on a watch glass.

The thus obtained solid materials were investigated by PXRD and the results are summarized in table 1.

| Solid Form | Solvents |
|---|---|
| Crystalline form according to WO 2021/092292 A1 | *tert*-butanol, ethanol, 1-propanol, 2-propanol, 1-butanol, 2-butanol, *iso*butanol, 1-pentanol, 2-pentanol, tert-amylalcohol, sec-amylalcohol, 1-hexanol, cyclopentanol, benzylalcohol, trifluoroethanol, ethylengycol, butanone, cyclohexanone, 1,2-dichloroethane, chloroform, anisole, toluene, xylene, acetonitrile, valeronitrile, benzonitrile, 1,4-dioxane, 1,2-dimethoxyethane, cyclopropyl methyl ether, heptane, diisopropyl ether, methyl *tert*-butyl ether, nitromethane, mesitylene, dimethyl sulfoxide, *N*,*N* dimethylformamide, *N*,*N*-dimethylacetamide, *N*-methyl-2-pyrrolidone, cyrene, sulfolane, methyl acetate, ethyl acetate, methyl benzoate, *n*-butyl acetate, *iso*-propyl acetate, ethyl propionate, *iso*-butyl acetate, dimethyl carbonate, diethyl carbonate, diethanolamine, triethylamine |
| | org. solvent/water mixtures (95/5, v/v): methanol, ethanol, 1-propanol, 2-propanol, 1-butanol, 2-butanol, *tert*-butanol, trifluoroethanol, acetonitrile, diethyl carbonate |
| Form HyA | methanol |
| Amorphous | acetone, 4-methyl-2-pentanone, dichloromethane, 1,2-dichlorobenzene, formic acid |

| | |
|---|---|
| **Table 1:** Summary of solid form screening outcome | |

As can be seen from table 1 the vast majority (91%) of the experiments lead to the same crystalline form as disclosed in WO 2021/092292 A1, 5 experiments gave amorphous avacopan and only a single experiment yielded Form HyA of the present invention.

### Example 2: Preparation of crystalline Form HyA seed crystals

Amorphous avacopan (50 mg) was dissolved in methanol (1 mL) upon slight heating. The clear solution was allowed to cool down to 2-8°C and stored for 20 hours at 2-8°C in a fridge. The crystals were collected by filtration to yield avacopan Form HyA.

### Example 3: Preparation of crystalline Form HyA

Amorphous avacopan (505 mg) was dissolved in methanol (10 mL) upon slight heating. To the clear solution Form HyA seed crystals (prepared according to Example 2 herein) were added and the solution was allowed to cool down to 2-8°C and stored for 20 hours at 2-8°C in a fridge. The crystals were collected by filtration and dried under vacuum at room temperature for 20 hours to yield 345 mg of avacopan Form HyA.

### Example 4: Preparation of crystalline Form HyA

Amorphous avacopan (3.027 g) was suspended in methanol (25 mL) and the obtained suspension was heated to 60°C. Additional methanol (30 mL) was added and the thus obtain clear solution was cooled to 0°C in 1 hour. During the cooling step Form HyA seed crystals (prepared according to Example 2 herein) were added at 50°C (seeds dissolved) and again at 13°C to initiate crystallization. The obtained suspension was further stirred for about 20 hours at 0°C. The crystals were collected by filtration, washed with cold methanol (10 mL) and dried under vacuum (about 5 mbar) at room temperature for 3 hours to yield 2.475 g of avacopan Form HyA.

### Example 5: Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

The reflection list of crystalline Form HyA of avacopan of the present invention is provided in Table 2 below.

**Table 2: Reflection positions of crystalline Form HyA of avacopan in the range of from 2 to 30° 2-Theta; a typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 3.7 | 14.0 | 18.5 | 23.0 |
| 6.1 | 14.3 | 19.0 | 23.2 |
| 7.4 | 14.8 | 19.2 | 23.5 |
| 8.9 | 15.1 | 19.4 | 24.5 |
| 9.8 | 15.6 | 19.5 | 24.9 |
| 10.5 | 15.9 | 20.0 | 25.2 |
| 11.1 | 16.1 | 20.4 | 25.6 |
| 12.1 | 16.6 | 20.6 | 26.2 |
| 12.3 | 16.9 | 21.0 | 26.5 |
| 12.5 | 17.1 | 21.8 | 27.1 |
| 12.7 | 17.4 | 22.0 | 28.6 |
| 13.4 | 17.8 | 22.4 | 29.3 |

### Example 6: Differential scanning calorimetry (DSC)

Crystalline form HyA of avacopan was investigated by DSC, which was performed on a Mettler Polymer DSC R instrument. The sample (4.21 mg) was heated in a 40 microliter aluminium pan with a pierced aluminium lid from 25 to 250°C at a rate of 10 K/min. Nitrogen (purge rate 50 mL/min) was used as purge gas.

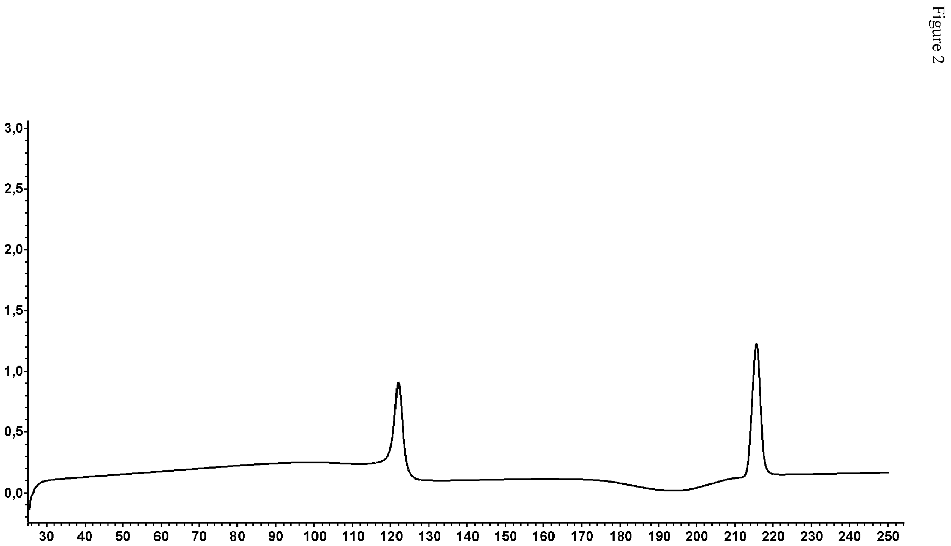

The DSC curve (see Figure 2) of the crystalline Form HyA of the present invention shows a first endotherm with an onset temperature of about 120°C, which is due to a melting process after previous dehydration. A broad exotherm with an onset temperature of about 176°C indicates a recrystallization event. Finally, the crystals melt at an onset temperature of about 213°C.

**Table 3: Thermal events of crystalline Form HyA during DSC experiment**

| **A vacopan** | **Thermal events** |
|---|---|
| Form HyA | endotherm: Tₒₙₛₑₜ: 119.6°C, Tₚₑₐₖ: 121.7°C |
| | exotherm: Tₒₙₛₑₜ: 175.9°C, Tₚₑₐₖ: 213.2°C |
| | endotherm: Tₒₙₛₑₜ: 213.2°C, Tₚₑₐₖ: 215.2°C |

### Example 7: Thermogravimetric analysis

TGA was performed on a Mettler TGA/DSC 1 instrument. The sample (5.24 mg) was heated in a 100 microliter aluminium pan closed with an aluminium lid from 25 to 250°C at a rate of 10 K/min. The lid was automatically pierced at the beginning of the measurement. Nitrogen (purge rate 50 mL/min) was used as purge gas.

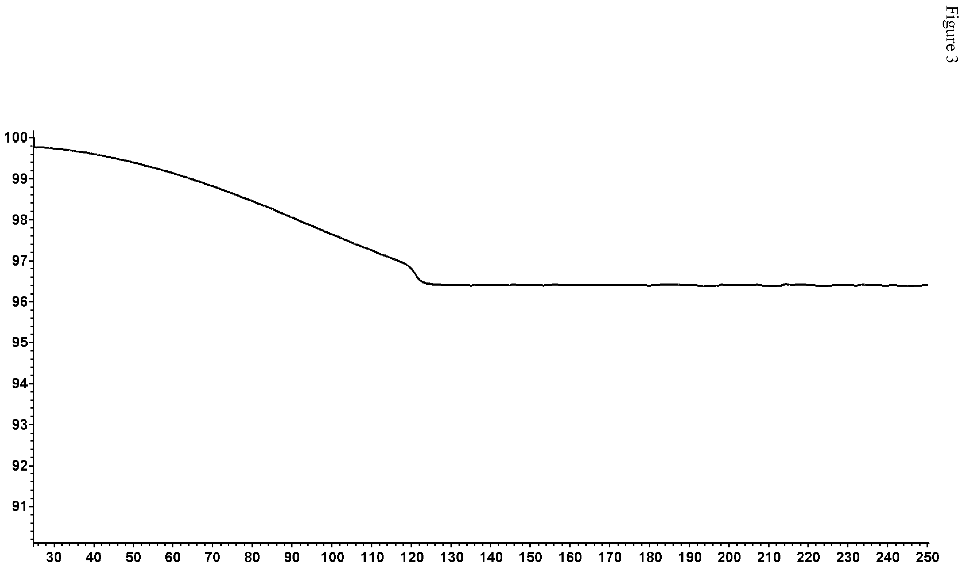

The TGA curve (see Figure 3) of the crystalline Form HyA of the present invention shows a mass loss of about 3.3% from the beginning of the measurement until about 130°C, which corresponds to the release of about 1.1 mol equivalents of water.

**Table 4: Mass loss during TGA experiment of avacopan Form HyA**

| **Avacopan** | **Mass loss** |
|---|---|
| Form HyA | 3.3 w-% until 130°C |

### Example 8: Gravimetric moisture sorption

Moisture sorption isotherms were recorded with an SPSx-1µ moisture sorption analyzer (ProUmid, Ulm) together with other samples. The measurement cycle was started at ambient relative humidity (RH) of 40%. RH was then decreased to 5% in 5% steps, followed by a further decrease to 3% and to 0%. Afterwards RH was increased from 0% to 90% in a sorption cycle and subsequently decreased to 0 % in a desorption cycle each in 5% steps. Finally, RH was increased to ambient relative humidity of 45% in 5% steps. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C.

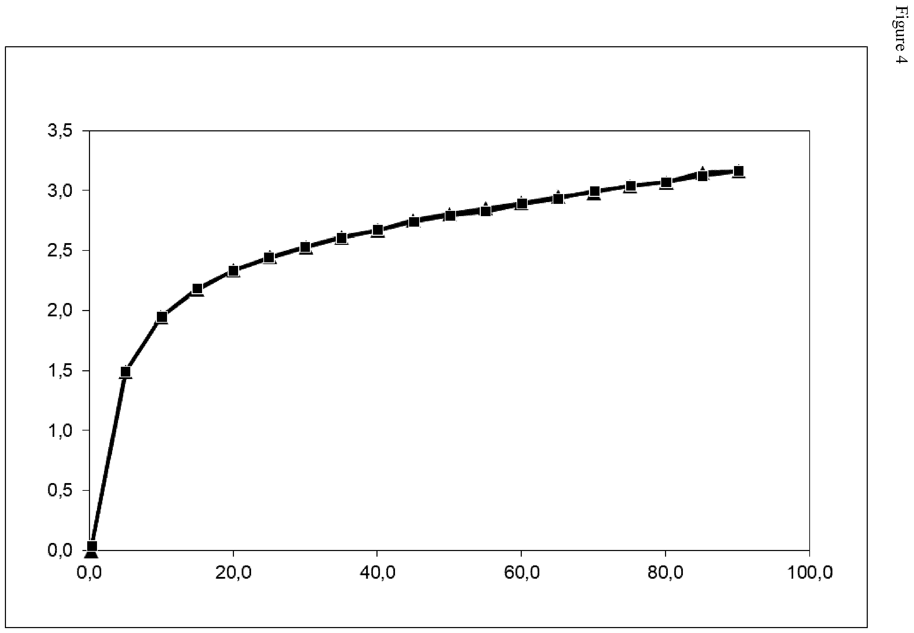

As can be seen from Figure 4 the actual water content depends on the relative humidity of the surrounding atmosphere. Thereby, water is taken up and released equally fast which is indicated by a missing hysteresis between the sorption and desorption curves. In the range of 15-90% RH the mass change is only 1.0%, hence in this range Form HyA can be assigned as being slightly hygroscopic. Only at very dry conditions *e.g*. below 15-0% RH humidity further 2.2% of water are released. This process is reversible and water is taken up again as soon as humidity increases again. The already mentioned missing hysteresis between the sorption and desorption curves, also indicates that no structural changes appear during the experiment. This assumption is strengthened by the fact that the sample still shows the same PXRD after the experiment.

**Table 5: Mass changes during GMS experiment with crystalline Form HyA of avacopan**

| **mass change (0-15% RH)** | **mass change (15-90%)** | **mass change (0-90%RH)** |
|---|---|---|
| 2.2 w-% | 1.0 w-% | 3.2 w-% |

### Comparative Example 1: Dissolution rates of crystalline avacopan forms in 0.1N HCl

Powder dissolution experiments were carried out in 0.1N HCl at 37°C for the crystalline form of avacopan disclosed in WO 2021/092292 A1 and the crystalline Form HyA of avacopan of the present invention. Particle sizes of the forms were kept as similar as possible to allow for proper comparison. The respective concentrations were determined by HPLC at a wavelength of 243 nm.

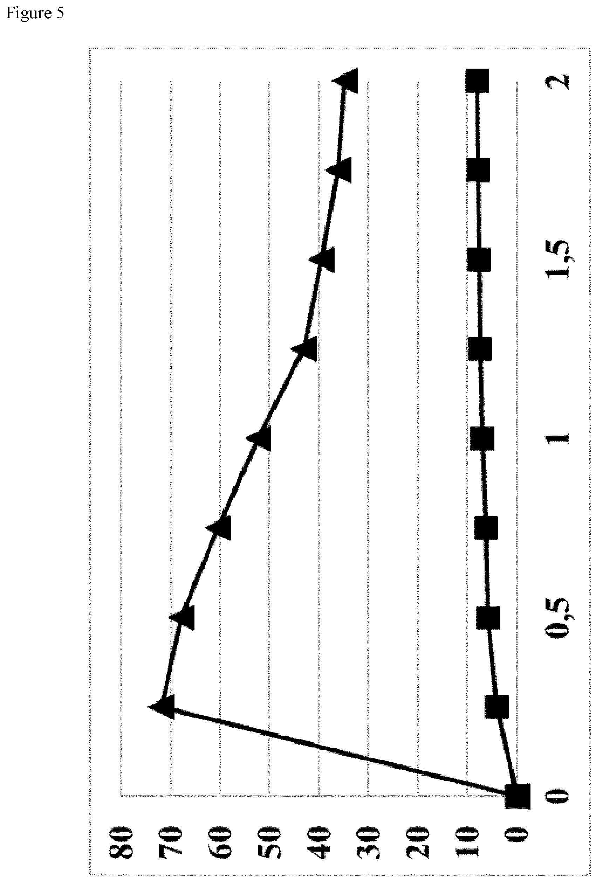

As can be seen from Table 6 and Figure 5 herein avacopan Form HyA of the present invention exhibits a significantly increased solubility in 0.1N HCl over the whole range from 15 minutes to 2 hours compared to the crystalline form of avacopan disclosed in WO 2021/092292 A1.

**Table 6: Dissolution profiles of crystalline avacopan forms in 0.1N HCl at 37°C**

| **Time [h]** | **Concentration in µg/mL** | |
|---|---|---|
| | **Form HyA of present invention** | **Crystalline Form of** WO 2021/092292 A1 |
| **0** | **0** | **0** |
| **0.25** | **72** | **4** |
| **0.5** | **68** | **6** |
| **0.75** | **60** | **6** |
| **1** | **52** | **7** |
| **1.25** | **43** | **7** |
| **1.5** | **40** | **8** |
| **1.75** | **36** | **8** |
| **2** | **35** | **8** |

## Claims

1. A crystalline form of avacopan (Form HyA) according to the chemical structure as depicted in Formula (A) 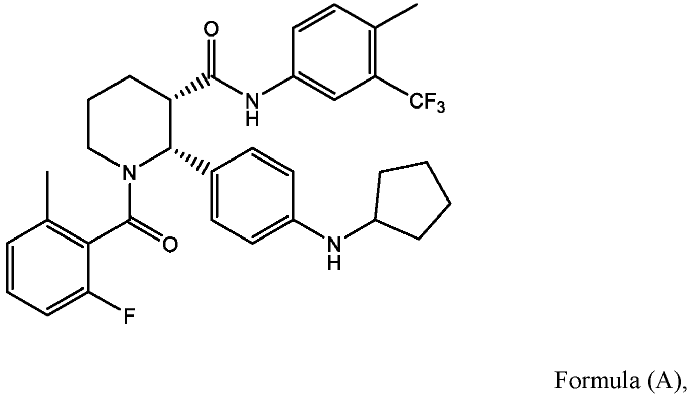 **characterized by** having a powder X-ray diffractogram comprising reflections at 2-Theta angles of (3.7 ± 0.2)°, (6.1 ± 0.2)° and (17.4 ± 0.2)°, when measured at a temperature in the range of from 20 to 30°C with Cu-Kalpha_{1,2} radiation having a wavelength of 0.15419 nm.

2. The crystalline form of claim 1 **characterized by** having a powder X-ray diffractogram comprising additional reflections at 2-Theta angles of (8.9 ± 0.2)° and/or (11.1 ± 0.2)°, when measured at a temperature in the range of from 20 to 30°C with Cu-Kalpha_{1,2} radiation having a wavelength of 0.15419 nm.

3. The crystalline form according to any one of the preceding claims **characterized by** having a differential scanning calorimetry curve comprising an endothermic peak, having an onset at a temperature of (120 ± 5)°C, when measured at a heating rate of 10 K/min.

4. A process for the preparation of the crystalline form (Form HyA) of avacopan as defined in any one of the preceding claims comprising:
(a) dissolving avacopan in methanol;
(b) crystallizing avacopan from the solution obtained in (a);
(c) separating at least a part of the crystals obtained in (b) from the mother liquor; and
(d) drying the crystals obtained in (c).

5. Use of the crystalline form (Form HyA) of avacopan as defined in any one of claims 1 to 3 for the preparation of a pharmaceutical composition comprising avacopan.

6. The use according to claim 5, wherein the pharmaceutical composition is an oral dosage form.

7. The use according to claim 6, wherein the oral dosage form is a capsule, a tablet or a suspension.

8. The use according to claim 7, wherein the oral dosage form is a hard gelatin capsule.

9. A pharmaceutical composition comprising the crystalline form (Form HyA) of avacopan as defined in any one of claims 1 to 3 and at least one pharmaceutically acceptable excipient.

10. The pharmaceutical composition of claim 9, wherein the crystalline form (Form HyA) of avacopan as defined in any one of claims 1 to 3 is present in an amount in the range of from 10 mg to 60 mg.

11. The pharmaceutical composition according to claim 9 or 10, wherein the pharmaceutical composition is an oral dosage form.

12. The pharmaceutical composition according to claim 10, wherein the oral dosage form is a capsule, a tablet or a suspension.

13. The pharmaceutical composition according to claim 11 or 12, wherein the oral dosage form is a hard gelatin capsule.

14. The crystalline form (Form HyA) of avacopan as defined in any one of claims 1 to 3 or the pharmaceutical composition of claims 8 to 13 for use as a medicament.

15. The crystalline form (Form HyA) of avacopan as defined in any one of claims 1 to 4 or the pharmaceutical composition of claims 8 to 13 for use in the treatment of anti-neutrophil cytoplasmic antibody (ANCA)-associated vasculitis (AAV).
